(19) Europäisches Patentamt
European Patent Office
Office européen des brevets

(11) Publication number: 0 286 418 B1

# (12) EUROPEAN PATENT SPECIFICATION

(45) Date of publication of patent specification: 08.07.92 (51) Int. Cl.$^5$: A61K 9/50, A61K 31/70

(21) Application number: 88303144.5

(22) Date of filing: 08.04.88

## (54) Anti-retroviral agents and delivery systems for the same.

(30) Priority: 10.04.87 US 37178
05.04.88 US 177788

(43) Date of publication of application:
12.10.88 Bulletin 88/41

(45) Publication of the grant of the patent:
08.07.92 Bulletin 92/28

(84) Designated Contracting States:
AT BE CH DE FR GB IT LI LU NL SE

(56) References cited:
EP-A- 0 196 185
WO-A-87/01283
WO-A-87/01284

CHEMICAL ABSTRACTS, vol.92, no.21, 26 May 1980, Columbus, OH (US); P.FURMANSKI et al., p.49, no.174458v

CHEMICAL ABSTRACTS, vol.86, no.9, 28 February 1977, Columbus, OH (US); E.MAYHEW et al., pp.28-29, no.50643h

CHEMICAL ABSTRACTS, vol.104, no.14, 07 April 1986, Columbus, OH (US); S.G.NORLEY et al., pp.391-392, no.115992h

(73) Proprietor: THE UNITED STATES OF AMERICA as represented by the Secretary, United States Department of Commerce
National Technical Information Service, Office of Government Inventions and Patents, 5285 Port Royal Road
Springfield, Virginia 22161(US)

(72) Inventor: Weinstein, John N.
9000 Rockville, Pike Building 10, Room 4b-54
Bethesda, MD 20892(US)

(74) Representative: Daley, Michael John et al
F.J. CLEVELAND & COMPANY 40/43 Chancery Lane
London, WC2A 1JO(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

### BACKGROUND OF THE INVENTION

#### Technical Field

The present invention relates to retroviruses and a method of preventing or treating diseases caused by retroviruses. More particularly, the present invention relates to human immunodeficiency virus (HIV) which has been implicated in acquired immunodeficiency syndrome (AIDS), new anti-HIV agents and a vehicle for the delivery of the new anti-HIV agents.

#### State of the Art

Acquired immunodeficiency syndrome (AIDS) has been traced to a retrovirus previously known as HTLV-III, LAV or ARV but recently re-named "human immunodeficiency virus" (HIV). The major immunological pathology caused by AIDS is a reduction in the number of CD4-positive (synonymous with "T4-positive") T-lymphocytes. Because CD4-positive lymphocytes are involved in almost all immune processes, and because other immune system cell types are also affected, it is not surprising that infection with the AIDS virus devastates immune function. The result is that patients infected with the virus become susceptible to lethal opportunistic infections, cancers and a variety of other illnesses. Currently, there is no treatment which does more than delay the progression of the disease. Without effective therapy, AIDS is known to be 100% fatal.

Azidothymidine (AZT) is one of the anti-HIV agents believed to affect the progression of HIV infection. However, AZT has certain drawbacks and limited utility which will become clear.

Rideout et al (EP-A-0196185) teaches a composition comprising phosphorylated forms of 3'-azido-3'-deoxythymidine for use in treating human retrovirus infections. Mitsuya et al (WO-A-87/01284) and Mitsuya et al (WO-A-87/01283) D2 teach compositions comprising 2' ,3'-dideoxyinosine; 2',3'-dideoxyguanosine; 2',3'-dideoxyadenosine and 2',3'-dideoxycytosine for use in inhibiting in vitro infectivity of HIV virus.

Norley et al (Journal of Immunology) D4 teaches the in vitro use of immunoliposomes containing either ododeoxyuridine (IUDR) or acyclovir (ACV) to treat herpes simplex virus (HSV) infected cells.

Mayhew et al (Chemical Abstract) tests ara-C or I $\beta$ arabinofuranosylcytosine 5'-triphosphate encapsulated in phospholipid vesicals for their effectiveness as inhibitors of SV4O-transformed 3T3 cells in vitro and the effectiveness of vesical-entrapped ara-C in mice to treat SV40 virus.

### SUMMARY OF THE INVENTION

It is therefore, an object of the present invention to provide new and effective anti-retroviral agents.

It is a further object of the present invention to provide new and effective anti-retroviral agents encapsulated in liposomes and compositions made therefrom.

It is still a further object of the present invention to provide an effective delivery system for targeting the anti-retroviral agents to the sites of actual or potential retroviral activity in the infected host.

It is an additional object of the present invention to provide a chemotherapeutic method of treating or preventing AIDS.

According to the present invention there is provided an anti-retroviral composition, characterised in that it comprises an effective amount of a chain-terminator phosphorylated nucleoside lacking a 3'-hydroxyl moiety encapsulated in liposomes to inhibit reverse transcriptase activity of a retrovirus, and a pharmaceutically acceptable carrier.

According to the present invention there is also provided a use in a method for the production of a medicament capable of inhibiting reverse transcriptase activity of a retrovirus, of an anti-retroviral composition characterised in that it comprises an effective amount of a chain-terminator phosphorylated nucleoside lacking a 3'-hydroxyl moiety encapsulated in liposomes.

Other objects and advantages of the present invention will become apparent from the present disclosure.

### BRIEF DESCRIPTION OF THE DRAWINGS

These and other objects, features and many of the attendant advantages of the invention will be better understood upon a reading of the following specification when considered in connection with the accompanying drawings wherein:

Figure 1 shows liposomes containing 40 mM ddCTP (top) and liposomes containing phosphate buffered saline (bottom). Electron micrographs (x75,000-133 Angstroms/mm) show images negatively stained with sodium phosphotungstate.

Figure 2 shows the size profile of a typical ddCTP liposome preparation.

Figure 3 shows the time course of p24 expression after infection of elutriated monocytes with human immunodeficiency virus. Bars represent standard errors of the mean (S.E.M.) of quadruplicate microtiter plate wells.

Figure 4 shows the time course of p24 levels on days 5, 7, 10 and 14 after treatment of HIV-infected human monocytes with liposomes containing 40 mM ddCTP (LD). Bars represent S.E.M. of quadruplicate microtiter plate wells.

Figure 5 shows the separation of free antibody from liposome-bound antibody using Sepharose 4B gel filtration chromatography. The first $^{125}I$ peak, centered at fraction 11, represents bound IgG.

## DETAILED DESCRIPTION OF PREFERRED EMBODIMENTS OF THE INVENTION

The above and various other objects and advantages of the present invention are achieved by a unique method of delivering anti-retroviral agents to the loci of retroviral activity and an anti-retroviral composition, comprising an effective amount of a chain-terminator phosphorylated nucleoside encapsulated in liposomes, to inhibit multiplication of retrovirus by blocking activity of retroviral reverse transcriptase activity, and a pharmaceutically acceptable carrier.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned hereunder are incorporated herein by reference. Unless described otherwise, all techniques used herein are standard methodologies well known to one of ordinary skill in the art.

The terms "anti-retroviral" agent, drug, preparation, composition and the like as used herein means that said agent, preparation, composition and the like is capable of controlling or inhibiting the proliferation or multiplication of the retrovirus in a susceptible host, the most important among the retrovirus pathogens being the HIV.

The term "chain-terminator phosphorylated nucleoside" or "chain-terminator nucleotide" as used herein means that the compound lacks a 3′-hydroxyl moiety, whereby the attachment of another nucleotide to the 3′ position of said phosphorylated nucleoside is blocked, resulting in the termination of retrovirus DNA chain elongation under the influence of reverse transcriptase (RT).

The anti-retroviral agents of the present invention have the formula:

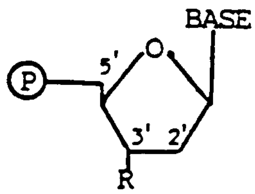

(1)

wherein the "Base" is selected from the group consisting of adenine, guanine, cytosine, thymine and analogues, derivatives or salts thereof which can be incorporated into growing DNA chains;

R is H, azido or a group which blocks the attachment of another nucleotide, preventing DNA chain elongation of the retrovirus under the influence of reverse transcriptase; and

P represents either mono, di, tri or other phosphates.

It is preferred that the compound of the general formula (1) be a 2′-deoxy nucleotide. Representative examples of such chain-terminator phosphorylated (triphosphates being preferred) nucleosides are as follows:

(a) azidothymidine 5′-triphosphate
(b) 2′,3′-dideoxycytidine-5′-triphosphate
(c) 2′,3′-dideoxyadenosine-5′-triphosphate
(d) 2′,3′-dideoxythymidine-5′-triphosphate
(e) 2′,3′-dideoxyguanosine-5′-triphosphate.

P may also represent other phosphorus-containing moieties including ones more metabolically stable than the phosphate. Representative examples are the methylphosphonates and phosphorothioates.

Contrary to what may be assumed otherwise, it is significant to note here that non-phosphorylated nucleosides per se, are inferior as anti-retroviral agents in the delivery systems described herein compared to the corresponding phosphorylated compounds. Thus, unphosphorylated azidothymidine, for instance, which is presently available as an anti-retroviral agent, is inferior and actually leaks out from the liposomes.

In contrast, the phosphorylated, particularly triphosphate, derivatives of chain-terminator nucleosides, are more efficacious and superior for liposomal preparations in accordance with the present invention for at least three reasons which are set forth hereunder.

(a) The triphosphates are much more water-soluble than the nucleosides per se and thus remain encapsulated in liposomes much longer ($t_{1/2}$ >60 hours at 4°C for leakage from liposomes described in Example 1, infra). It was discovered, in fact, that azidothymidine is released from liposomes rapidly ($t_{1/2}$ <2 hours at 4°C for leakage from similar liposomes) and hence is a poor candidate for liposomal delivery.

(b) The triphosphates do not pass across cell membranes easily, hence they are not as useful as free drugs. However, this fact confers advantages in selectivity if they could be transported into the cell by liposomes, because the molecules of the phosphorylated drug released from liposomes into the cytoplasm of a cell would not readily escape into the external medium, and any drug released from the liposome extracellularly would not readily enter the cells non-specifically.

(c) The triphosphate is the form actually recognized and incorporated into the growing viral DNA chain by viral RT. Therefore, nucleoside analogs which enter the cell in free form must first be phosphorylated before they can function to terminate the chain. Since various other metabolic pathways compete with phosphorylation, it is clear that a molecule of nucleoside, entering the cell cytoplasm, which still needs to be phosphorylated would be less potent against virus than a molecule which has already been phosphorylated.

It should be understood, however, that even if the liposomes are taken up by endocytosis, it cannot be a priori assumed that the anti-viral agent encapsulated in the liposome will be released and become available in its active form to the cell cytoplasm so as to act as a chain-terminator.

Furman et al, 1986 PNAS, 83: 8333-7, report that the triphosphate derivative of AZT binds to the HIV RT. However, as has been mentioned herein, free forms of triphosphorylated AZT or other such compounds do not enter the cells readily. Hence, they cannot be as effective as "drugs".

Based on the properties and characteristics noted above, it is quite clear that without actual tests, the superiority and advantages of phosphorylated chain-terminator nucleosides relative to non-phosphorylated compounds could not have been a priori deduced or predicted. Furthermore, nothing in the prior art could have suggested the actual anti-retroviral efficacy of a liposomal preparation made from the phosphorylated chain-terminator nucleosides without actual tests having been performed.

As is well known, liposomes can be formed by any of a large number of standard techniques, and may be quite large (up to about 100 μm) or quite small (down to about 25 nm). The size influences both the mechanisms of interaction with cells and the biodistribution. For example, liposomes smaller than about 80 nm can enter cells through coated pits after binding via a ligand to a receptor or antigen on the cell surface. Thus, smaller liposomes would be preferred if a ligand is to be attached. On the other hand, phagocytosis will be favored if the liposome is larger. In terms of biodistribution, liposomes smaller than about 100 nm can pass through sinusoidal clefts in organs such as the liver, spleen, and bone marrow, whereas larger particles cannot.

Of course, liposomes can also be formed with many different compositions. Most are based on two-chain phospholipids such as the phosphatidylcholines and sphingomyelins. Liposomes may also include other lipids such as cholesterol (often added to confer "toughness" on the lipid structures), stearylamine (for positive charge), and various other lipids for negative charge. The most commonly added negative lipids are phosphatidylglycerol, phosphatidylserine, diacetylphosphate, and cardiolipin. Negative charge tends to increase the avidity of liposome uptake by phagocytic cells. Phosphatidylserine, in particular, has been reported to constitute a particular signal for recognition by phagocytes and also to play a role in activation of those cells. Thus, phosphatidylserine may be preferred for inclusion in liposomes when the targeted cells are phagocytic or endocytic.

Typically, liposome formation and encapsulation of water-soluble substances involves dispersing the lipid, adding a solution containing the drug, and ensuring that the lipid is effectively hydrated by the solution. It is generally necessary to carry out these steps above the phase transition(s), if any, of the lipid mixture. Alternative approaches to liposome formation involve dispersion of lipid in an organic solvent, addition of drug-containing aqueous solution to form a colloidal suspension, and removal of the organic

solvent to leave liposomes containing the drug. After any of these procedures, the liposomes could be reduced to small size by processes such as sonication, extrusion through a small orifice, or extrusion through a filter with holes of the appropriate size. Unencapsulated or free drug is usually removed from the external solution by column chromatography, centrifugation, dialysis, or the like. Of course, liposomes can also be lyophilized and loaded with drug after formation.

The following examples illustrate the preferred embodiments of the present invention employing dideoxycytidine triphosphate as the anti-retroviral agent. It is emphasized that ordinarily such compounds would not be thought of as effective "drugs" since they do not enter the cells as easily as the unphosphorylated forms; however, such compounds are effective drugs against retroviruses when delivered through liposomes according to the present invention.

## Example 1

### Formation of Liposomes containing dideoxycytidine triphosphate

The materials used for formation of liposomes were as follows: dipalmitoyl phosphatidylcholine (DPPC) and dipalmitoyl phosphatidylglycerol (DPPG) were obtained from Avanti Biochemicals (Birmingham, Al., USA), primary standard cholesterol from Eastman Kodak (Rochester, N.Y., USA), $^{14}C$-cholesteryl oleate (i.e., cholesteryl-[1]$^{14}C$-oleate] from New England Nuclear (Billerica, Mass., USA) and 2′,3′-dideoxycytidine-5′-triphosphate (ddCTP) from Pharmacia (Piscataway, N.J., USA).

DPPC (62.5 mg), DPPG (12.5 mg), cholesterol (21.3 mg), and $^{14}C$-cholesteryl oleate (0.125 $\mu$ Ci) were dried out of chloroform onto the walls of a glass vial, under a stream of nitrogen gas. Final removal of chloroform was achieved by several hours on a freeze-dryer under high vacuum. Solution for encapsulation was made up by hydrating 60 micromoles of dideoxycytidine triphosphate (ddCTP) with 0.6 ml of sterile distilled water and 0.6 ml of sterile phosphate-buffered saline. The final ddCTP concentration was 40 mM. One ml of ddCTP solution was then added to the glass vial, which was flushed with nitrogen gas to exclude oxygen. The vial was heated to 45° C, and maintained at that temperature for 15 minutes, with intermittent vortex-mixing (5×15-second periods). The liposome suspension was then transferred to a plastic cryotube and subjected to 5 freeze-thaw cycles using liquid nitrogen and a 37° C water bath. The suspension was forced under 200-700 pounds per square inch of pressure through an Extruder (Lipex Biomembranes, Inc., Vancouver, B.C., Canada) fitted with two 0.4-micron polycarbonate filters. This extrusion process was performed 10 times, always at about 45° C (above the transition temperature of the lipids). "Empty" liposomes were made identically except that phosphate buffered saline was substituted for the ddCTP solution. In the case of ddCTP-liposomes, free drug was removed by dialysis overnight against two changes of phoshpate buffered saline. In other, similar experiments, free ddCTP was removed by size exclusion column chromatography.

Liposomes containing the other preferred chain-terminator phosphorylated nucleosides are prepared in a similar manner.

### Characterization of liposomes

The amount of lipid in the preparation was determined from $^{14}C$ counts ($^{14}C$-cholesteryl oleate) in a standard beta counting scintillation fluid. The amount of ddCTP was assessed from the optical density at 280 nm. Samples for optical density reading were prepared by adding 0.9 ml of 65:25 chloroform ethanol solution to 0.5 ml of the sample. This eliminated light scatter that would otherwise have resulted from the presence of lipid particles. Negative staining electron microscopy was performed using glow-discharged formvar-carbon coated grids and sodium phosphotungstate. Release of ddCTP from liposomes was assessed by dialysis of samples against phosphate buffered saline. The integrity of ddCTP was assessed by high-performance gradient-elution anion exchange chromatography.

### Toxicity and anti-viral effect in vitro:

Liposomes were tested for toxic effect on two types of cells which chronically produce HIV. One clone (8.E6) was derived from the lymphoid cell line A.301; the other clone (U.1) was derived from the promonocytic line U937. Cells ($1\times10^6$/well) in RPMI medium with 10% fetal calf serum were pipetted into a flat-bottom 96-well microtiter plate. To each well was added 50 microliters of solution containing ddCTP-liposomes, empty liposomes, free ddCTP or phosphate buffered saline. Fifty microliters of phorbol myristyl acetate (PMA) was then added to each well to a final concentration of $10^{-9}$ M for stimulation, or else 50

microliters of saline was added for non-stimulated configurations. The plates were incubated at 37°C in 7% $CO_2$ for about 48 hours. 100 microliters of supernatant was then taken from each well to analyze for reverse transcriptase (RT) activity using polyadenylate and oligo-deoxythymidine. To perform the RT assay, 10 microliters of supernatant containing RT was added to a cocktail containing poly A, oligo dT (Pharmacia, Inc. Piscataway, N.J., USA) $MgCl_2$, and $^{32}$P-dTTP (Amersham) and incubated for 2 hours at 37°C. Ten microliters of the cocktail was then spotted onto DE81 paper, dried, and washed in 1X saline sodium citrate buffer. The paper was then dried, cut and counted on a Beckman LS 7000 scintillation counter.

Cells in the remaining 100 microliters in each well were resuspended vigorously, and 50 microliter samples were then transferred to corresponding wells in a new plate. To each well in the new plate, 50 microliters of fresh medium and 20 microliters of $^{3}$H-thymidine were added. The plates were incubated at 37°C in 7% $CO_2$ for about 4 hours, harvested, washed, and counted for uptake of $^{3}$H-thymidine.

Characterization of liposomes

The above procedure trapped 9% of the total ddCTP, and the calculated capture was 2.6 microliters/micromole lipid. The $T_{1/2}$ for leakage at 4°C was >60 hours. Negative staining electron-microscopy showed liposomes of varying sizes ranging to a maximum of about 0.4 µm (Fig. 1). The ddCTP was about 99% pure by HPLC prior to use.

Toxicity:

Tritiated thymidine uptake was used to assess toxic effects of ddCTP liposomes on the lymphoid (8.E6) and promonocytic (U.1) clones. The results are shown in Table 1. No significant toxic effects were seen for either cell type with free ddCTP, empty liposomes, or ddCTP-liposomes, regardless of whether or not the cells were stimulated with PMA. As judged by trypan blue exclusion test, the cells remained more than 90% viable at all doses.

TABLE 1.

LACK OF TOXICITY OF ddCTP-LIPOSOMES ASSESSED BY $^3$H-THYMIDINE UPTAKE IN TWO CELL TYPES

| | | U.1 (promonocytic) | | 8.E6 (lymphocytic) | |
|---|---|---|---|---|---|
| | Dose | -PMA* | +PMA | -PMA | +PMA |
| No drug: | 0.0** | 100 | 80 | 100 | 85 |
| ddCTP-liposomes: | 10.0 | 100 | 80 | 80 | 85 |
| | 1.0 | 107 | 84 | 99 | 102 |
| | 0.1 | 90 | 77 | 111 | 85 |
| | 0.01 | 118 | 77 | 112 | 87 |
| ddCTP: | 10.0 | 96 | 75 | 162 | 107 |
| | 1.0 | 101 | 105 | 100 | 96 |
| | 0.1 | 79 | 99 | 112 | 97 |
| | 0.01 | 90 | 99 | 115 | 78 |
| Empty liposomes: | 10.0 | 80 | 85 | 83 | 88 |
| | 1.0 | 83 | 90 | 119 | 113 |
| | 0.1 | 82 | 115 | 99 | 115 |
| | 0.01 | 115 | 87 | 84 | 117 |

* PMA: phorbol myristic acetate

** ddCTP dose in micrograms/ml (or equivalent in empty liposome lipid). Each entry represents the mean of duplicate determinations, relative to the control with no drug and no stimulation.

Anti-viral Effect

Table 2 shows the results for RT assays on 8.E6 and U.1 cells in the absence of PMA stimulation. ddCTP-liposomes substantially inhibited RT activity at all doses tested, whereas ddCTP and empty liposomes had much less effect.

TABLE 2.

INHIBITION OF HIV REVERSE TRANSCRIPTASE ACTIVITY IN U.1 CELLS BY LIPOSOMES CONTAINING DIDEOXYCYTIDINE TRIPHOSPHATE

| Dose* | ddCTP-Lip | Empty Lip | Free ddCTP |
|---|---|---|---|
| 10.0 | 29 +/- 8%** | 87 +/- 6% | 69 +/- 21% |
| 1.0 | 19 +/- 2 | 82 +/- 17 | 61 +/- 4 |
| 0.1 | 27 +/- 10 | 80 +/- 15 | 80 +/- 12 |
| 0.01 | 26 +/- 0.2 | 68 +/- 38 | 67 +/- 19 |

* ddCTP dose in micrograms/ml (or equivalent in empty liposome lipid)

** Percentages relative to controls with no drug (mean of duplicate determinations, ±standard deviation). The aggregate mean for ddCTP-liposomes differs significantly from that for empty liposomes ($p<.01$) and that for free ddCTP ($p<.01$) using two-tail Student's t-test and proportional error model.

Table 3 shows the results for RT assays on U.1. cells stimulated by $10^{-9}$M PMA. Substantiated inhibition is seen at all levels of liposomes containing ddCTP.

TABLE 3.

INHIBITION OF HIV REVERSE TRANSCRIPTASE ACTIVITY IN PMA-STIMULATED U.1 CELLS BY LIPOSOMES CONTAINING DIDEOXYCYTIDINE TRIPHOSPHATE

| Dose* | ddCTP-Lip | Empty Lip | Free ddCTP |
|---|---|---|---|
| 10.0 | 53%** | ND | 75% |
| 1.0 | 55 | 96 | 75 |
| 0.1 | 58 | 93 | 96 |
| 0.01 | 51 | 110 | 84 |

* ddCTP dose in micrograms/ml (or equivalent in empty liposome lipid)

** Percentages normalized by average of empty liposome controls. Each entry is the mean of duplicate determinations. In this experiment cells were simulated with 1 nM PMA. Aggregate mean for ddCTP-liposomes differs significantly from that for empty liposomes ($p<0.01$) and that for free ddCTP ($p<0.01$) using two-tail Student's t-test and proportional error model.

ND = Not done.

Liposomes containing dideoxycytidine triphosphate for toxicity testing in vivo.

Large multilamellar liposomes were prepared as described above, but with the following changes: The composition was DPPC (150 mg), DPPG (40 mg), and cholesterol (60 mg). The ddCTP (40 micromoles) was hydrated in sterile distilled water and added to the lipid. The suspension was flushed with argon gas, heated to 50 ° C, and vortex-mixed intermittently over a two-hour period. The suspension was then sonicated for 5-8 minutes using a cup-horn sonicator (Heat Systems-Ultrasonics, Inc., Plainview, N.Y., USA). This procedure trapped approximately 35% of the ddCTP, as determined by elution on a size-exclusion column. Empty liposomes were made identically by substituting phosphate buffered saline for the ddCTP solution. For in vivo experiments, free drug was not removed.

For toxicity testing, C57BL/6J female (18-19 gm) mice were used. Starting on day 0, the mice (5 per group) were injected i.v. under pentobarbital anesthesia every 2-3 days for 3 weeks with ddCTP liposomes. The injectate contained about 718 micrograms of ddCTP (approx. 35% entrapped) and 12.5 mg lipid per injection. Other groups of five mice received phosphate buffered saline. Some of the mice were infected on day zero by i.p. injection of LP-BM5 murine leukemia virus (Mosier et al, J. Exp. Med. 161:766-784 (1985)).

However, this had no independent effect on weight or survival during the time of the experiment. Results shown in Table 4 show no significant toxicity attributable to the ddCTP liposomes with respect to weight profiles or mortality. Other groups of 5 mice (not shown) injected with 1/5 and 1/25 the dose of ddCTP liposomes showed similar lack of toxicity.

TABLE 4.

LACK OF TOXICITY OF ddCTP LIPOSOMES INJECTED INTO MICE

| | Infected animals | | |
|---|---|---|---|
| Day* | ddCTP-Lip** | Saline | Saline |
| 0 | 18.7 +/- 0.9 | 17.9 +/- 1.3 | 18.0 +/- 1.6 |
| 2 | 18.9 +/- 0.6 | 19.2 +/- 1.5 | 19.1 +/- 1.6 |
| 4 | 19.3 +/- 0.8 | 19.3 +/- 1.4 | 19.2 +/- 1.4 |
| 6 | 19.6 +/- 0.8 | 19.0 +/- 1.3 | 19.0 +/- 1.1 |
| 8 | 20.2 +/- 0.8 | 16.9 +/- 1.4 | 19.3 +/- 1.4 |
| 13 | 20.2 +/- 0.9 | 19.2 +/- 1.4 | 19.5 +/- 1.3 |
| 19 | 20.8 +/- 0.8 | 19.1 +/- 1.7 | 19.9 +/- 1.5 |
| 22 | 21.1 +/- 0.6 | 19.5 +/- 1.7 | 20.0 +/- 1.5 |
| Net change: | +2.4 | +1.6 | +2.0 |
| Survivors: | 5/5 | 5/5 | 5/5 |

* Day after injection and infection (n = 5 per group).

** Each injectate contained 12.5 mg total lipid, 251 micrograms of encapsulated ddCTP, and 467 micrograms of free ddCTP in 50 microliters. Animals were injected every 2-3 days. No significant differences in weight curves were seen. Under the conditions of the experiment, infection was not expected to influence weight significantly.

Example 2

Liposomes for Inhibition of the Human Immunodeficiency Virus in Monocytes

Preparation of Liposomes

Egg yolk phosphatidylcholine (EPC) and bovine brain phosphatidylserine (PS) were obtained from Avanti Biochemicals (Birmingham, Ala., USA). Thin layer chromatography (elution with chloroform:methanol:water 65:25:4) revealed no visible impurities with loads of 200 micrograms. Cholesterol, $^{14}C$-cholesteryl oleate, and ddCTP were obtained from the same sources as in Example 1. The ddCTP was found to be greater than 98% pure (with respect to O.D. 268) by elution on a VYDAC 303NT405 nucleotide

HPLC column (Separations Group, Hesperia, Calif., USA). Partially purified 5,6-di$^3$H-2′,3′-dideoxycytidine-5′-triphosphate ($^3$H-ddCTP) was obtained from Moraveck Biochemicals (Brea, California, USA). Portions of the tritiated stock were repurified on the nucleotide column prior to use.

EPC (56.3 mg), PS (28.8 mg), cholesterol (33.4 mg), and $^{14}$C-cholesteryl oleate (0.125 micro Ci) were dried out of chloroform onto the walls of a glass vial under a stream of nitrogen gas. Liposomes containing either 4 or 40 mM ddCTP were prepared by hydration, vortex-mixing, freeze-thaw cycling, and extrusion through polycarbonate filters as described in Example 1 except that $^3$H-ddCTP (usually 2 micro Ci) was added to the ddCTP stock to serve as a radioactive tracer. Unencapsulated ddCTP was removed by gel filtration column chromatography or by centrifugation of small columns containing Sephadex G25M (Pharmacia), following standard procedures such as described by Fry, et al., Anal. Biochem. 90:809-815, 1978.

Characterization of Process

The amounts of lipid and ddCTP in the preparation were calculated from $^{14}$C and $^3$H counts, respectively. Encapsulation was generally in the range of 1.5 - 2 microliters/micromole EPC. The size distribution of liposomes was determined by quasi-elastic light-scattering (QELS) using an N4MD multiple-angle submicron particle size analyzer (Coulter, Hialeah, Fla., USA). Figure 2 shows the size distribution for a typical preparation that had been stored at 4° C in phosphate-buffered saline for about 2 weeks. The profile is substantially similar to that of a fresh preparation. The weight average diameter was about 215 nm with the coefficient of variation being about 100 nm.

The release of liposome contents as a function of time was assessed by centrifugal elution of free ddCTP through Centrifree filters (Amicon, Danvers, Mass., USA). Typically, 100 microliters of suspension was centrifuged at 1500 G for about 10 minutes, 100 microliters additional phosphate-buffered saline was added, and the filter was again spun for about 10 minutes. Pooled filtrate was counted for $^3$H-ddCTP to determine released drug, and the filter itself was similarly counted to establish the mass balance. Very little $^{14}$C-cholesteryl oleate was ever found in the filtrate, indicating that liposomal lipid was not passing through the filter. These studies, as well as dialysis experiments such as described in Example 1, indicate very little leakage under the conditions of routine liposome storage (at 4° C, in phosphate-buffered saline). Leakage tests were also conducted under conditions mimicking those of the viral experiments infra, i.e., in tissue culture plates with 96 flat-bottom wells (Costar, Cambridge, Mass., USA) at 37° C in a $CO_2$ incubator in Dulbecco's Minimal Eagles Medium supplemented with gentamycin, glutamine, and 10% fetal calf serum (DMEM-GG-FCS). The results are shown in Table 5. Very little leakage of ddCTP was observed over a period of 72 hours, either in the presence or in the absence of adherent monocytes. In contrast, 50% of the ddC was released within 1 hour, and 95% within 4 hours. These results clearly demonstrate that the nucleoside itself (ddC) is not as suitable for encapsulation in liposomes as is the phosphorylated form.

TABLE 5.

LEAKAGE OF ddCTP AND ddC FROM LIPOSOMES AT 37°C, IN THE PRESENCE AND ABSENCE OF ADHERENT HUMAN MONOCYTES

| | % of total drug in liposomes | | | |
|---|---|---|---|---|
| time (h) | ddCTP (cells) | ddCTP | ddC (cells) | ddC |
| 0 | 84.4 | 85.6 | 55.7 | 58.5 |
| 0.16 | 83.8 | 74.3 | 60.4 | 58.7 |
| 0.32 | 84.4 | 78.5 | 50.9 | 48.7 |
| 1 | 78.0 | 77.2 | 32.2 | 30.3 |
| 2 | 83.3 | 82.1 | 19.2 | 23.0 |
| 4 | 82.9 | 78.5 | 14.4 | 8.6 |
| 24 | 75.9 | 81.7 | 9.5 | 7.5 |
| 48 | 68.0 | 75.7 | 4.8 | 6.4 |
| 72 | 78.4 | 87.4 | 8.0 | |

Effect of liposomes on viability and activity of human monocytes

Human peripheral blood mononuclear cells were obtained by standard procedures employing continuous flow cell separation (leukapheresis), such as described in Wahl, et al., Cellular Immunol. 85:373-383 (1984). The preparation was diluted in phosphate-buffered saline and layered onto gradients of Ficoll-Paque (Pharmacia-PL Biochemicals, Piscataway, N.J., USA). After centrifugation, mononuclear cells were pipetted from the interface, washed, and suspended in DMEM-GG-FCS. They were then fractionated by countercurrent centrifugal elutriation. The procedure comprises the Beckman elutriation system including a J-21 Beckman Centrifuge, a JE-6 elutriator rotor, a stroboscope, and a Masterflex pump and pumphead. The cells were loaded into the rotor and fractionated by elution at increasing flow rates. Fractions 7 and 8, highly enriched for monocytes, were pooled for use.

Empty liposomes, ddCTP-liposomes, ddCTP, and ddCTP plus empty liposomes were all tested for their effect on three different monocyte functions: (i) percent lysis of Candida albicans; (ii) production of prostaglandin $E_2$; and (iii) production of superoxide (Wahl, et al., Cellular Immunol. 85:384-395 (1984). No consistent effects were found for empty liposomes, ddCTP-liposomes, free ddCTP, or ddCTP plus empty liposomes. Table 6 shows the lack of effect on superoxide generation. Phorbol myristyl acetate (PMA) served as a positive control. There was no demonstrable effect on trypan blue entry or cell numbers as a consequence of the incubations.

TABLE 6

SUPEROXIDE GENERATION BY MONOCYTES TREATED WITH ddCTP LIPOSOMES, EMPTY LIPOSOMES, FREE ddCTP, and ddCTP PLUS EMPTY LIPOSOMES

Superoxide (nmoles/well)

| ddCTP (uM) | ddCTP-Lip | Empty Lip | Free ddCTP | ddCtP + Lip |
|---|---|---|---|---|
| Control | 0.62 (.08) | 0.71 (.12) | 0.89 (.12) | 0.89 (.13) |
| 0 | 1.11 (.06) | 1.08 (.05) | 1.19 (.06) | 1.46 (.03) |
| .018 | 0.99 (.04) | 1.24 (.04) | 1.33 (.05) | 1.27 (.08) |
| .09 | 1.19 (.11) | 1.17 (.10) | 1.42 (.10) | 1.27 (.08) |
| .45 | 1.02 (.05) | 1.27 (.13) | 1.33 (.08) | 1.23 (.06) |
| 2.2 | 0.83 (.07) | 1.27 (.05) | 1.21 (.02) | 1.81 (.13) |
| 11 | 1.21 (.08) | 0.99 (.02) | 0.85 (.04) | 1.35 (.06) |
| 56 | 1.34 (.04) | 1.14 (.04) | 0.77 (.04) | 1.60 (.10) |
| PMA | 3.23 (.09) | 3.30 (.12) | 3.26 (.05) | 3.46 (.12) |

PMA: phorbol myrystyl acetate treatment (positive control). Standard error of the mean in parentheses.

Uptake of lipid and ddCTP by monocytes

Uptake of $_3$H-deoxyctidine triphosphate ($^3$H-dCTP) was used as an indicator of the uptake of vesicle contents by cells. Uptake experiments were done by plating 5 × $10^6$ monocytes in each well of a 6-well microtiter plate (Costar) and adding reagents one or two days later. Incubations were done in DMEM-GG-FCS at 37°C. The rate of increase of dCTP content in the cells over a 9-hour period (Table 7) averaged 0.29 pmole/million cells/hr with liposomes containing 4 mM EPC. The uptake of $^{14}$C lipid marker is shown in Table 8. Other experiments established that ddCTP and ddCMP are not readily taken up by the cells (Table 9).

TABLE 7

UPTAKE OF LIPOSOME-ENCAPSULATED dCTP BY ADHERENT HUMAN MONOCYTES

| Time (h) | pmole dCTP/1 million cells |
|---|---|
| 1 | 1.25 |
| 3 | 1.33 |
| 6 | 2.04 |
| 9 | 2.62 |

0.1 mM EPC, 24.3 uM dCTP, 4 mM dCTP inside liposomes, 2.43 mmole dCTP/mole EPC

TABLE 8

UPTAKE OF LIPOSOMES BY ADHERENT HUMAN MONOCYTES AT 37°C

| Time (h) | EPC concentration pmole lipid/1 million cells | |
|---|---|---|
| 1 | 8.0 | 15.0 |
| 3 | 7.2 | 16.9 |
| 6 | 10.0 | 29.9 |
| 9 | 11.3 | 36.6 |
| 20 | 13.6 | 51.9 |

Tests were conducted in 10% FCS under conditions mimicking those of studies on anti-viral activity.

TABLE 9.

UPTAKE OF DIDEOXYNUCLEOTIDES BY ADHERENT HUMAN MONOCYTES AT 37°C

| | pmole drug/1 million cells | | | | | | | | | |
|---|---|---|---|---|---|---|---|---|---|---|
| | ddCTP | | | ddCMP | | | ddC | | | ddC+DPM |
| Time(h) | mean | SD | SE | mean | SD | SE | mean | SD | SE | |
| 0. | 0.15 | 0.21 | 0.15 | 0.07 | 0.02 | 0.01 | 0.05 | 0.05 | 0.03 | |
| 0.33 | 0.09 | 0.07 | 0.05 | 0.18 | 0.00 | 0.00 | 0.01 | 0.01 | 0.01 | |
| 1.00 | 0.09 | 0.10 | 0.06 | 0.22 | 0.16 | 0.09 | 0.19 | 0.05 | 0.03 | 0.11 |
| 3.00 | 0.11 | 0.11 | 0.06 | 0.08 | 0.04 | 0.02 | 0.43 | 0.17 | 0.10 | 0.13 |
| 6.00 | 0.33 | 0.08 | 0.05 | 0.20 | 0.01 | 0.01 | 1.30 | 0.06 | 0.03 | 0.26 |
| 9.00 | 0.43 | | | | | | 1.90 | | | 0.51 |
| 20.00 | 0.66 | | | | | | 0.81 | | | 0.27 |

The concentration of drugs was 1 µM.

DPM = 100 µM dipyridamole

The means, SD and SE, are calculated from 3 replicates.

The morphology of uptake was assessed by growing cells in microscope slide chambers and incubating with liposomes made as described above but with approximately 1 mole percent fluorescent lipid added. The fluorescent lipid was either N-(lissamine rhodamine B sulfonyl)-phosphatidylethanolamine or N-fluorescein-phosphatidyl-ethanolamine. After incubation with liposomes at 37° C, the cells showed surface-staining and also showed internal fluorescent aggregates suggestive of endocytosed material.

Stability of ddCTP in serum-containing medium

When free ddCTP was placed in DMEM-GG-FCS under conditions mimicking the anti-viral tests, it was largely converted to ddCMP within 2 hours and was 23% ddC in 6 hours (Table 10). ddCMP placed in the medium was 93% converted to ddC within 72 hours (Table 11). When the same incubations were done in the absence of monocytes, the results were essentially identical. These findings demonstrate that liposomes can protect the drug from metabolic conversion which would be inevitable if the drug were added to the medium in free form.

TABLE 10

METABOLISM OF ddCTP at 37°C IN 10% FETAL CALF SERUM

| | Percent of total drug $^3H$ | | | | | |
|---|---|---|---|---|---|---|
| | ---Without Cells--- | | | --With Cells- | | |
| Time (h) | ddCTP | ddCMP | ddC | ddCTP | ddCMP | ddC |
| 0.5 | 39.6 | 38.8 | 13.6 | 67.8 | 32.2 | 0 |
| 0.75 | 20.3 | 75.4 | 3.8 | 30.2 | 64.5 | 1.7 |
| 1.5 | 19.5 | 75.4 | 2.6 | 14.4 | 73.2 | 6.5 |
| 4 | 2.7 | 84.3 | 11.5 | 1.4 | 86.6 | 12 |
| 6 | 0.6 | 78.5 | 20.7 | 1.4 | 75.3 | 23.1 |

Studies were conducted under conditions mimicking those of anti-viral tests. ddCMP: dideoxycytidine monophosphate. Data points represent integrals under HPLC peaks from gradient elution on a VYDAC nucleotide column.

TABLE 11

METABOLISM OF ddCTP at 37°C IN 10% FETAL CALF SERUM

| | Percent of total drug $^3H$ | | | | | |
|---|---|---|---|---|---|---|
| | ---Without Cells--- | | | --With Cells- | | |
| Time (h) | ddCTP | ddCMP | ddC | ddCTP | ddCMP | ddC |
| 0.5 | 0 | 99.2 | 0.8 | 0 | 99.7 | 0.3 |
| 0.75 | 0 | 99.7 | 0.3 | 3.1 | 99.9 | 0 |
| 1.5 | 0 | 98.5 | 1.5 | 1.3 | 98.1 | 0.6 |
| 4 | 0 | 89.9 | 10.1 | 0 | 92.0 | 8.0 |
| 6 | 0 | 66.3 | 24.8 | 0 | 70.2 | 29.8 |
| 72 | 0 | 7.7 | 92.3 | 0 | 7.3 | 92.7 |

Studies were conducted under conditions mimicking those of anti-viral tests. ddCMP: dideoxycytidine monophosphate. Data points represent integrals under HPLC peaks from gradient elution on a VYDAC nucleotide column.

Inhibition of HIV proliferation in monocytes

Elutriated monocytes were infected (day 0) with a monocyte/macrophage-derived strain of HIV-1 (Ba-L), washed, and allowed to settle in plastic tissue culture wells. They were then treated on days 1, 3, 5 and 7 with ddCTP-liposomes, empty liposomes, free ddCTP, or empty liposomes plus free ddCTP. Samples of supernatant were collected on days 3, 5, 7, 10, 14, 17 and 21 and inactivated with Triton X100® detergent. The samples were assayed for HIV p24 antigen using an ELISA assay (DuPont, Wilmington, DE, USA). The antibody-coated plates were incubated with samples at room temperature (about 22-25° C overnight (about 16 hours), and the second layer detection step was done at 37° C for 1 hour. In order to read concentrations in the non-linear range of the assay, OD 490 nm readings were taken as a function of time after addition of the substrate, as well as after stopping the reaction with sulfuric acid. Control experiments indicated infection of the cells, accompanied by increasing supernatant concentrations of p24 (Figure 3).

Figure 4 shows the results for ddCTP liposomes. ddCTP liposomes inhibited p24 expression by >90% at 62.5 nM drug concentration. Empty liposomes had little or no effect, even at 64-fold higher lipid concentrations. Hence, the lipid itself could not account for the observed activity. Free ddCTP and ddCTP plus liposomes were active at similar concentrations. The observation that the liposomes release <10% of their contents in 6 hours and <20% in 24 hours under the experimental conditions used is against the possibility that the drug was leaking out of the liposomes, converting over days to ddC, and being taken up in free form by the cells. If that were the mechanism of activity, one would have expected a significant delay in the effectiveness of the liposomal ddCTP. Such delay was not observed.

Thus, the data presented herein clearly demonstrate the efficacy of the liposomal preparations of the present invention against HIV without apparent side effects or toxicity. To further increase the selectivity of the liposomal preparation of the present invention, specific ligands including antibodies or antibody fragments either against CD4 or against coat polyprotein of the virus (whose epitopes are exposed on the surface of the host cell at least at some point during the viral replication cycle) conjugated with the liposomes can, of course, also be used. Furthermore, the viral coat protein or a fragment thereof such as the T-peptide or one of its analogues can be used as a ligand.

Techniques for preparing such conjugates are routine and well known in the art. When so made, the preparations of the present invention can be naturally targeted and delivered in an effective dose either to the endocytic and phagocytic cells which form a reservoir for the retrovirus or, if conjugated with a suitable ligand, can be targeted to the CD4-positive lymphocytes and the like. Example 3 is illustrative.

Example 3

Antibody-bearing ddCTP-liposomes

As mentioned above, antibodies can be used to target liposomes for interaction with particular cell types (Weinstein, et al., Biochim. Bophys. Acta 509:272-288 (1978). In the context of AIDS, it will be useful to target the CD4 and Class II antigens (as parts of the HIV binding site), gp120 then viral outer coat protein), gp41 (the viral inner coat protein), and other antigenic determinants characteristic of either the virus or the host cell. It will also be useful to target Fc receptors and other receptors responsible in monocytes and macrophages for uptake of opsonized particles. This may enhance the effectiveness of liposomes containing chain terminators in inhibiting viral growth in these cells. Hence, Fc receptors were targeted by attaching mouse IgG (including the Fc portion) to liposomes. Of course, it would also be possible to attach only the Fc domain or a portion thereof to serve the same purpose.

To produce antibody-bearing liposomes, a modification of the method described by Leserman, et al. (Nature 288:602-604 (1980)) was used. The antibody selected for the study was 36-7-5, a murine $IgG_{2a}$ reactive with $H\text{-}2K^k$. The object was to "opsonize" liposomes for uptake by Fc receptors on the monocyte lineage and other cell types.

Liposomes were made in essentially the same manner as in Example 2, but with the addition of 5 mole% N-[4-(p-maleimidophenyl)butyryl] phosphatidylethanolamine (MPB-PE; Matthay, et al., Ca Res. 44:1880-1886 (1984)) obtained from Avanti, Birmingham, Ala., USA. The final composition was EPC (48.5 mg), bovine PS (28.8 mg), cholesterol (33.4 mg), MPB-PE (9.5 mg), $^{14}C$-cholesteryl oleate (200 micro Ci). Free ddCTP was separated by gel filtration on a column of Sephadex G25 packed into a 1-ml syringe. A sample of 36-7-5 antibody was labled with $125_I$ by the standard Iodo-gen technique to a specific activity of $5.26 \times 10^{14}$ cpm/mmole. Antibody was then modified using 3-(2-pyridyldithio)propionate (SPDP; Pharmacia, Piscataway, N.J., USA). To 1 mg of antibody (0.01 mg $^{125}I$-IgG and 0.99 mg unmodified IgG) in 1 ml was added 1.6 microliters of 20 mM SPDP. The solution was vortex-mixed occasionally during a 30-minute

incubation at room temperature and eluted on a PD 10 column (Pharmacia) equilibrated with 200 mM acetate buffer at pH 4.5. Appropriate fractions for collection were indicated by counting the $^{125}I$. The dithiopyridyl groups introduced into the antibody were then reduced to yield thiol by addition of dithiothreitol at a final concentration of 50 mM. After about 20 minutes incubation at room temperature, the protein was separated from DTT by passage over a PD10 column pre-equilibrated with 10 mM Hepes-buffered saline, pH8.0. To couple antibody to liposomes, 50 microliters of liposome suspension (2.6 micromolar EPC) was added to 100 microliters of $^{125}I$-labeled thiolated antibody and 350 microliters of phosphate-buffered saline. The solution was incubated at room temperature with stirring for about 17 hours. The liposomes were then separated from free antibody (Figure 5) using a standard Sepharose 4B column (Pharmacia).

The calculated average number of antibody molecules per liposome (assuming an average diameter of about 250 nm) was 11. Approximately 75% of the antibody recovered was bound to liposomes.

For studies of uptake by the monocytes, cells were plated at $5 \times 10^6$ per well in 6-well plates (Costar). After one day, the solution was removed, and the cells were washed twice with DMEM-GG-FCS and once with phosphate-buffered saline. 1 ml of liposome-containing DMEM-GG-FCS was then added and the cells incubated at 37°C for 0, 1.5, 3, 6, 24 or 40 hours. 0.6 ml of 0.4% sodium dodecyl sulfate solution was added to each well, and the plates were shaken to remove cells from the plastic. The dissolved cells were then counted for $^{125}I$, $14_C$, and $3_H$.

Table 12 shows the results. As indicated by $14_C$ counts, the cells took up more of the antibody-bearing liposomes than of the liposomes not bearing the antibody.

TABLE 12

UPTAKE OF ANTIBODY-BEARING LIPOSOMES BY HUMAN MONOCYTES AT 37°C IN 10% FETAL CALF SERUM

| Time (h) | Picomole lipid/million cells: Plain Lipos | MPB-PE Lipos | Antibody-Lipos |
|---|---|---|---|
| 0 | 20.1 | 41.6 | 53.2 |
| 1.5 | 28.1 | 59.6 | 181.3 |
| 3.0 | 27.9 | 77.6 | 125.8 |
| 6.0 | 33.2 | 95.1 | 194.3 |
| 24.0 | 68.2 | 225.0 | 360.6 |
| 40.0 | 150.6 | 316.8 | 505.1 |

Tests were conducted under conditions mimicking those of anti-viral studies.

Example 4.

Other chain-terminator nucleosides and nucleotides encapsulated in liposomes for anti-viral effect

The properties of dideoxycytidine and dideoxycytidine triphosphate with respect to encapsulation in liposomes were described in Example 2. A number of other phosphorylated and unphosphorylated nucleosides have also been studied to determine their suitability for encapsulation in liposomes.

2′,3′-dideoxyguanosine 5′-triphosphate (ddGTP), 2′,3′-dideoxyadenosine 5′-triphosphate (ddATP), 2′,3′-dideoxythymidine 5′-triphosphate (ddTTP), 2′,3′-dideoxycytidine 5′-diphosphate (ddCDP), and 2′,3′-dideoxycytidine 5′-monophosphate (ddCMP) were obtained from Pharmacia (Piscataway, N.J., USA). Liposomes containing each of these agents were prepared using 56 mg EPC, 29 mg PS, 33 mg cholesterol, and 0.125

micro Ci $^{14}C$-cholesteryl oleate. Solutions for encapsulation were 8 mM nucleoside or nucleotide in phosphate-buffered saline pH 7. Liposomes were prepared essentially as described in Example 2, by drying lipid onto the walls of a glass vial, hydrating the lipid with a portion of the solution to be encapsulated, vortex-mixing, and freeze-thawing 5 times with liquid nitrogen. However, the lipid was hydrated in 0.8 ml, rather than 1.0 ml, and the extrusion step was eliminated. Hence, the product obtained was expected to be freeze-thawed multilamellar liposomes. The preparation was washed by centrifugation 3 times to remove unencapsulated solute. The volume encapsulated was determined by extraction, using equal volumes of chloroform:methanol (65:25) and phosphate buffered saline. The nucleoside and nucleotide solutes partitioned largely into the aqueous phase, whereas lipid was found in the organic phase and near the interface after centrifugation. The amount of solute encapsulated was determined by standard ultraviolet spectroscopy, and the lipid was measured by counting the $^{14}C$ label in scintillation fluid. The volumes of encapsulation obtained are listed in Table 13.

Rates of solute release were determined by adding 145 microliters of phosphate buffered saline to 25 microliters of liposomes and centrifuging in an Airfuge (Beckman) at approximately 100,000G for 10 minutes. The liposome pellets were then counted for $^{14}C$ lipid, and 100 microliters of each supernatant was diluted 5-fold with phosphate buffered saline for ultraviolet spectroscopy. Table 13 shows the release observed after approximately 2 days at 4° C. In each case, the triphosphates are released less, and the less phosphorylated forms are released to a greater extent. The largest release was observed for the non-phosphorylated form ddT.

The anti-viral effect of these compounds is determined as described herein supra.

Table 13

Volume of encapsulation and percent release at 4°C in 2 days for various chain-terminator nucleosides and nucleotides in liposomes

| Encapsulated Molecule | Volume of Encapsulation (microliters/micromole EPC) | Percent Release |
|---|---|---|
| ddGTP | 2.74 | 0.3% |
| ddATP | 1.30 | 0.9 |
| ddTTP | 1.61 | 0.04 |
| ddT | 1.29 | 45.1 |
| ddCDP | 1.26 | 6.2 |
| ddCMP | 0.76 | 6.9 |

EPC: Egg phosphatidylcholine; ddGTP: dideoxyguanosine triphosphate; ddATP: dideoxyadenosine triphosphate; ddTTP: dideoxythymidine triphosphate; ddT: dideoxythymidine; ddCDP: dideoxycytidine diphosphate; ddCMP: dideoxycytidine monophosphate

For the preparation of a pharmaceutical composition, any suitable non-toxic carriers, adjuvants, sterilants and the like can be used. Preferred examples of such additives are physiological saline, non-toxic sterile buffers, anti-bacterial agents and the like. Of course, the preparations of the present invention can be administered for treating an infected or susceptible host by any suitable route such as intravenous, subcutaneous, intramuscular and the like.

It is noted, however, that based on the evidence presented herein, certain critical factors had to be established in arriving at the present invention: first, that phosphorylated, chain-terminator nucleoside

analogues are more efficacious as anti-retroviral agents than the non-phosphorylated forms if present within the cell; second, that such phosphorylated, chain-terminator analogues can be encapsulated in liposomes, without leaching therefrom; and that the liposomes can be targeted to the endocytic cells which are a major reservoir of HIV infection. Once the feasibility of stable liposomal preparations was established, the next critical factor was to determine the efficacy of the liposomal preparations against HIV without causing serious deleterious side effects. The results presented herein for the first time provide a potent anti-HIV composition and an effective delivery system for the same.

## Claims

1. An anti-retroviral composition, characterised in that it comprises an effective amount of a chain-terminator phosphorylated nucleoside lacking a 3'-hydroxyl moiety encapsulated in liposomes to inhibit reverse transcriptase activity of a retrovirus, and a pharmaceutically acceptable carrier.

2. The composition of claim 1, further characterised in that said chain-terminator phosphorylated nucleoside is mono-, di- or tri-phosphate.

3. The composition of claim 1 or 2, further characterised in that the chain-terminator phosphorylated nucleoside is selected from azidothymidine triphosphate, dideoxycytidine triphosphate, dideoxyadenosine triphosphate, dideoxythymidine triphosphate, dideoxyguanosine triphosphate and mixtures thereof.

4. The composition of claim 3, further characterised in that said chain terminator is dideoxycytidine triphosphate.

5. The composition of any preceding claim, further characterised in that said liposomes are conjugated with a ligand for selective targeting of the liposomes.

6. The composition of claim 5, further characterised in that said ligand is an antibody or fragment thereof having specific binding affinity to CD-4 or coat polyprotein of the retrovirus.

7. The composition of claim 5 or 6, further characterised in that said ligand is retroviral envelope protein or a portion thereof.

8. The composition of any preceding claim, further characterised in that said retrovirus is human immunodeficiency virus.

9. The use in a method for the production of a medicament capable of inhibiting reverse transcriptase activity of a retrovirus, of an anti-retroviral composition characterised in that it comprises an effective amount of a chain-terminator phosphorylated nucleoside lacking a 3'-hydroxyl moiety encapsulated in liposomes.

10. The use of claim 9, further characterised in that said chain-terminator phosphorylated nucleoside is a mono-, di- or tri-phosphate.

11. The use of claims 9 or 10, further characterised in that the chain-terminator phosphorylated nucleoside is selected from azidothymidine triphosphate, dideoxycytidine triphosphate, dideoxyadenosine triphosphate, dideoxythymidine triphosphate, dideoxyguanosine triphosphate and mixtures thereof.

12. The use of claim 11, further characterised in that said chain terminator is dideoxycytidine triphosphate.

13. The use of any of claims 9 to 12, further characterised in that said liposomes are conjugated with a ligand for selective targeting of the liposomes.

14. The use of claim 13, further characterised in that said ligand is an antibody or fragment thereof having specific binding affinity to CD-4 or coat polyprotein of the retrovirus.

15. The use of any of claims 9 to 14, further characterised in that said ligand is retroviral envelope protein

or a portion thereof.

16. The use of any of claims 9 to 15, further characterised in that said retrovirus is human immunodeficiency virus.

## Revendications

1. Composition anti-rétrovirale, caractérisée en ce qu elle comprend une quantité efficace d'un nucléoside phosphorylé terminateur de chaîne, dépourvu de groupement hydroxyle en 3', encapsulé dans des liposomes pour inhiber l'activité de la transcriptase inverse chez un rétrovirus, et un support pharmaceutique acceptable.

2. Composition selon la revendication 1, caractérisée en ce que, en outre, ledit nucléoside phosphorylé terminateur de chaîne est un mono-, di-ou tri-phosphate.

3. Composition selon la revendication 1 ou 2, caractérisée en ce que, en outre, ledit nucléoside phosphorylé terminateur de chaîne est choisi parmi l'azidothymidine-triphosphate, la didésoxycytidine-triphosphate, la didésoxyadénosine-triphosphate, la didésoxythymidine-triphosphate, la didésoxyguanosine-triphosphate et leurs mélanges.

4. Composition selon la revendication 3, caractérisée en ce que, en outre, ledit terminateur de chaîne est la didésoxycytidine-triphosphate.

5. Composition selon l'une quelconque des revendications précédentes, caractérisée en ce que, en outre, lesdits liposomes sont associés à un ligand leur assurant une vectorisation spécifique vers leur cible.

6. Composition selon la revendication 5, caractérisée en ce que, en outre, ledit ligand est un anticorps ou un fragment d'anticorps présentant une affinité de liaison spécifique pour CD-4 ou une polyprotéine d'enveloppe du rétrovirus.

7. Composition selon la revendication 5 ou 6, caractérisée en ce que, en outre, ledit ligand est une protéine d'enveloppe rétrovirale ou une fraction de celle-ci.

8. Composition selon l'une quelconque des revendications précédentes, caractérisée en ce que, en outre, ledit rétrovirus est un virus de l'immunodéficience humaine.

9. Utilisation d'une composition anti-rétrovirale, pour préparer un médicament capable d'inhiber l'activité transcriptase inverse d'un rétrovirus, ladite composition anti-rétrovirale étant caractérisée en ce qu elle comprend une quantité efficace d'un nucléoside phosphorylé terminateur de chaîne, dépourvu de groupement hydroxyle en 3' et encapsulé dans des liposomes.

10. Utilisation selon la revendication 9, caractérisée en ce que, en outre, ledit nucléoside phosphorylé terminateur de chaîne est un mono-, diou tri-phosphate.

11. Utilisation selon la revendication 9 ou 10, caractérisée en ce que, en outre, le nucléoside phosphorylé terminateur de chaîne est choisi parmi l'azidothymidine-triphosphate, la didésoxycytidine-triphosphate, la didésoxyadénosine-triphosphate, la didésoxythymidine-triphosphate, la didésoxyguanosine-triphosphate et leurs mélanges.

12. Utilisation selon la revendication 11, caractérisée en ce que, en outre, ledit terminateur de chaîne est la didésoxycytidine-triphosphate.

13. Utilisation selon l'une quelconque des revendications 9 à 12, caractérisée en ce que, en outre, lesdits liposomes sont associés à un ligand leur assurant une vectorisation spécifique vers leur cible.

14. Utilisation selon la revendication 13, caractérisée en ce que, en outre, ledit ligand est un anticorps ou fragment d'anticorps présentant une affinité spécifique pour CD-4 ou une protéine d'enveloppe du rétrovirus.

15. Utilisation selon l'une quelconque des revendications 9 à 14, caractérisée en ce que, en outre, ledit ligand est une protéine d'enveloppe rétrovirale ou une fraction de celle-ci.

16. Utilisation selon l'une quelconque des revendications 9 à 15, caractérisée en ce que, en outre, ledit rétrovirus est un virus de l'immunodéficience humaine.

**Patentansprüche**

1. Anti-retrovirale Zusammensetzung, dadurch gekennzeichnet, daß sie eine zur Blockierung reverser Transkriptase-Aktivität eines Retrovirus wirksame Menge eines in Liposomen eingekapselten phosphorylierten Kettenabbruch-Nukleosids, bei welchem eine 3'-Hydroxyl-Hälfte fehlt, und einen pharmazeutisch annehmbaren Träger enthält.

2. Zusammensetzung nach Anspruch 1, dadurch gekennzeichnet, daß das phosphorylierte Kettenabbruch-Nukleosid Mono-, Di-oder Triphosphat ist.

3. Zusammensetzung nach Anspruch 1 oder 2, dadurch gekennzeichnet, daß das phosphorylierte Kettenabbruch-Nukleosid aus Azidothymidin-Triphosphat, Dideoxycytidin-Triphosphat, Dideoxyadenosin-Triphosphat, Dideoxythymidin-Triphosphat, Dideoxyguanosin-Triphosphat und Gemischen davon ausgewählt ist.

4. Zusammensetzung nach Anspruch 3, dadurch gekennzeichnet, daß das Kettenabbruch-Nukleosid Dideoxycytidin-Triphosphat ist.

5. Zusammensetzung nach einem der vorhergehenden Ansprüche, dadurch gekennzeichnet, daß die genannten Liposomen zum Erreichen einer selektiven Zielgerichtetheit der Liposomen mit einem Liganden konjugiert sind.

6. Zusammensetzung nach Anspruch 5, dadurch gekennzeichnet, daß der Ligand ein Antikörper oder ein Fragment hiervon mit spezifischer Bindungsaffinität zu CD-4 oder dem Mantel-Polyprotein des Retrovirus ist.

7. Zusammensetzung nach Anspruch 5 oder 6, daß der Ligand ein retrovirales Hüllprotein oder ein Teil hiervon ist.

8. Zusammensetzung nach einem der vorhergehenden Ansprüche, dadurch gekennzeichnet, daß der Retrovirus ein Human-Immundefekt-Virus ist.

9. Verwendung einer Anti-retroviralen Zusammensetzung in einem Verfahren zur Herstellung eines Medikaments zum Blockieren reverser Transkriptase-Aktivität eines Retrovirus, dadurch gekennzeichnet, daß die Zusammensetzung eine wirksame Menge eines in Liposomen eingekapselten phosphorylierten Kettenabbruch-Nukleosids enthält, bei welchem eine 3'-Hydroxyl-Hälfte fehlt.

10. Verwendung nach Anspruch 9, dadurch gekennzeichnet, daß das phosphorylierte Kettenabbruch-Nukleosid ein Mono-, Dioder Triphosphat ist.

11. Verwendung nach Anspruch 9 oder 10, dadurch gekennzeichnet, daß das phosphorylierte Kettenabbruch-Nukleosid aus Azidothymidin-Triphosphat, Dideoxycytidin-Triphosphat, Dideoxyadenosin-Triphosphat, Dideoxythymidin-Triphosphat, Dideoxyguanosin-Triphosphat und Gemischen hiervon ausgewählt ist.

12. Verwendung nach Anspruch 11, dadurch gekennzeichnet, daß das Kettenabbruch-Nukleosid Dideoxycytidin-Triphosphat ist.

13. Verwendung nach einem der Ansprüche 9 bis 12, dadurch gekennzeichnet, daß die genannten Liposomen zum Erreichen einer selektiven Zielgerichtetheit der Liposomen mit einem Liganden konjugiert sind.

14. Verwendung nach Anspruch 13, dadurch gekennzeichnet, daß der Ligand ein Antikörper oder ein Fragment hiervon mit spezifischer Bindungsaffinität zu CD-4 oder dem Mantel-Polyprotein des Retrovirus ist.

15. Verwendung nach einem der Ansprüche 9 bis 14, dadurch gekennzeichnet, daß der Ligand ein retrovirales Hüllprotein oder ein Teil hiervon ist.

16. Verwendung nach einem der Ansprüche 9 bis 15, dadurch gekennzeichnet, daß der Retrovirus ein Human-Immundefekt-Virus ist.

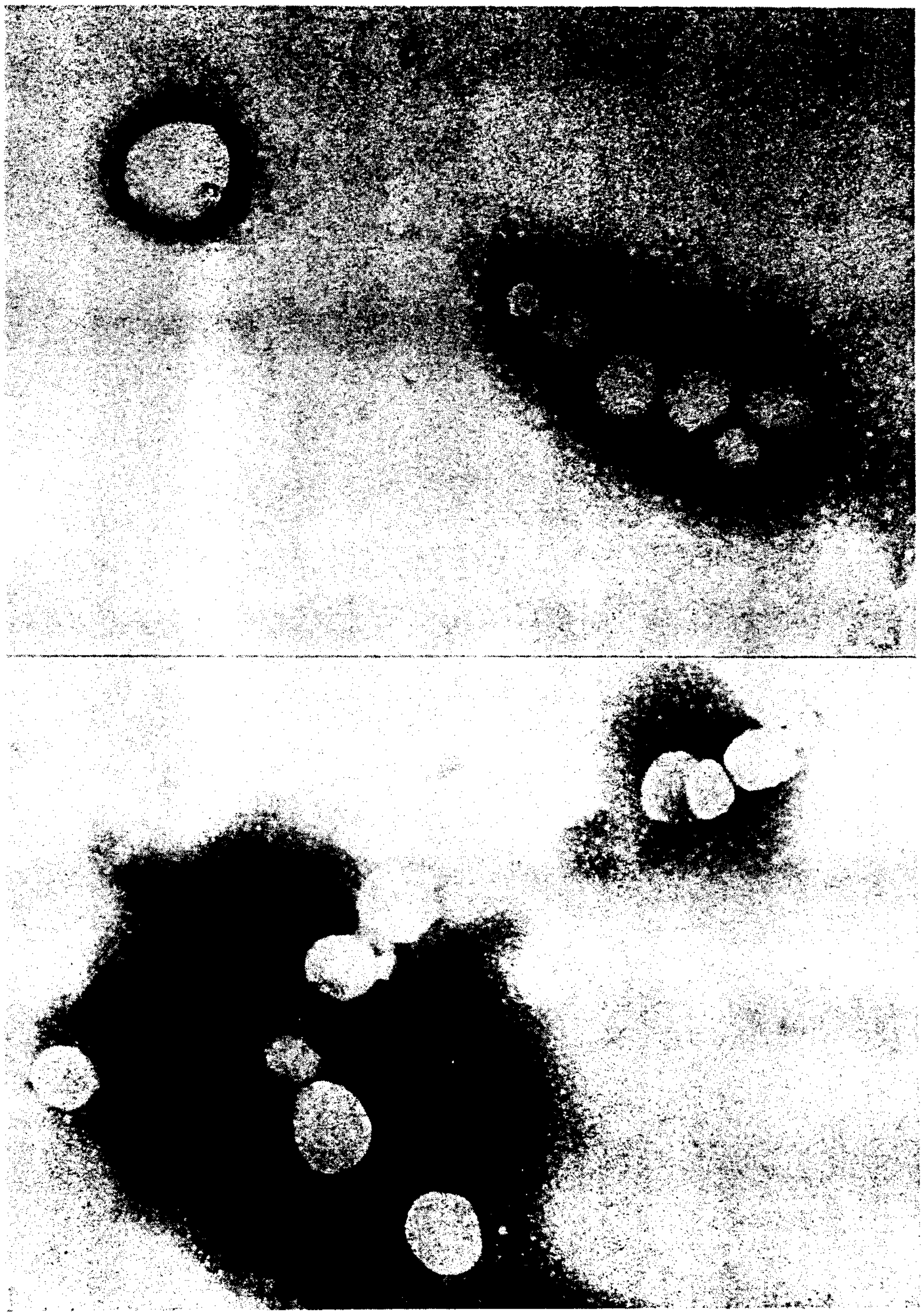

QELS SIZING OF ddCTP LIPOSOMES

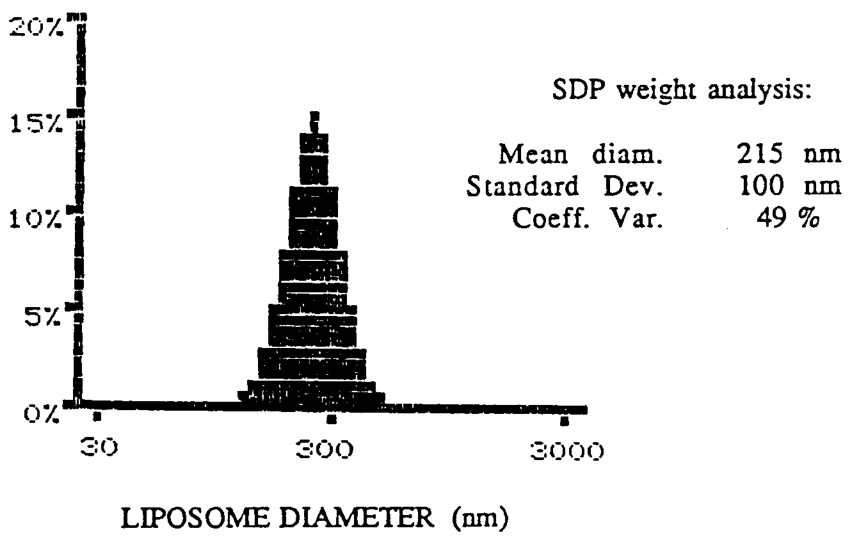


FIG.2

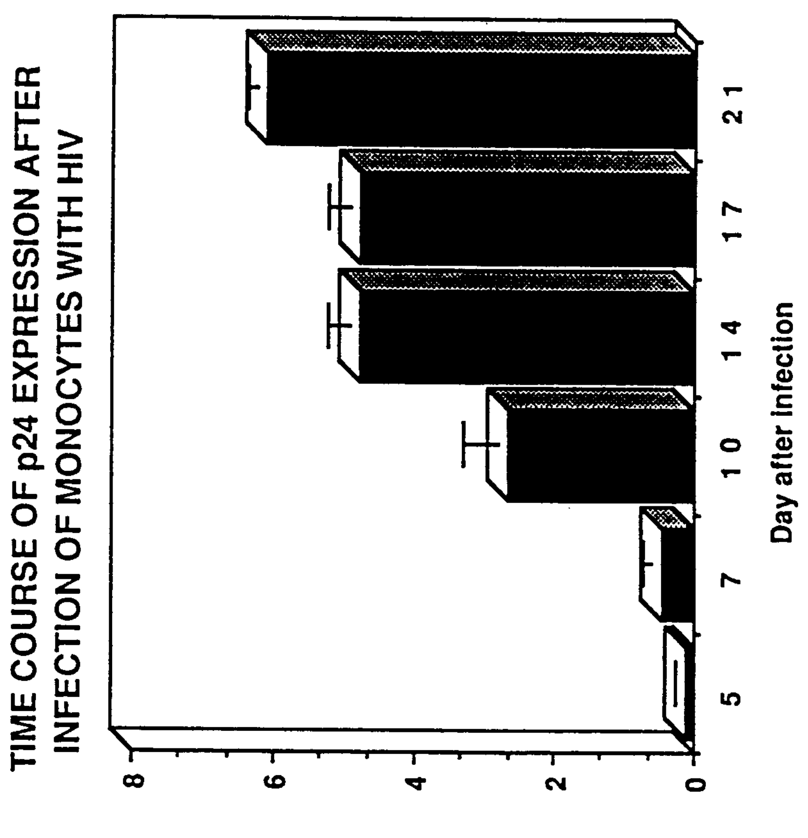
TIME COURSE OF p24 EXPRESSION AFTER INFECTION OF MONOCYTES WITH HIV
p24 (ng/ml)
0
2
4
6
8
5
7
1 0
1 4
1 7
2 1
Day after Infection

FIG.3

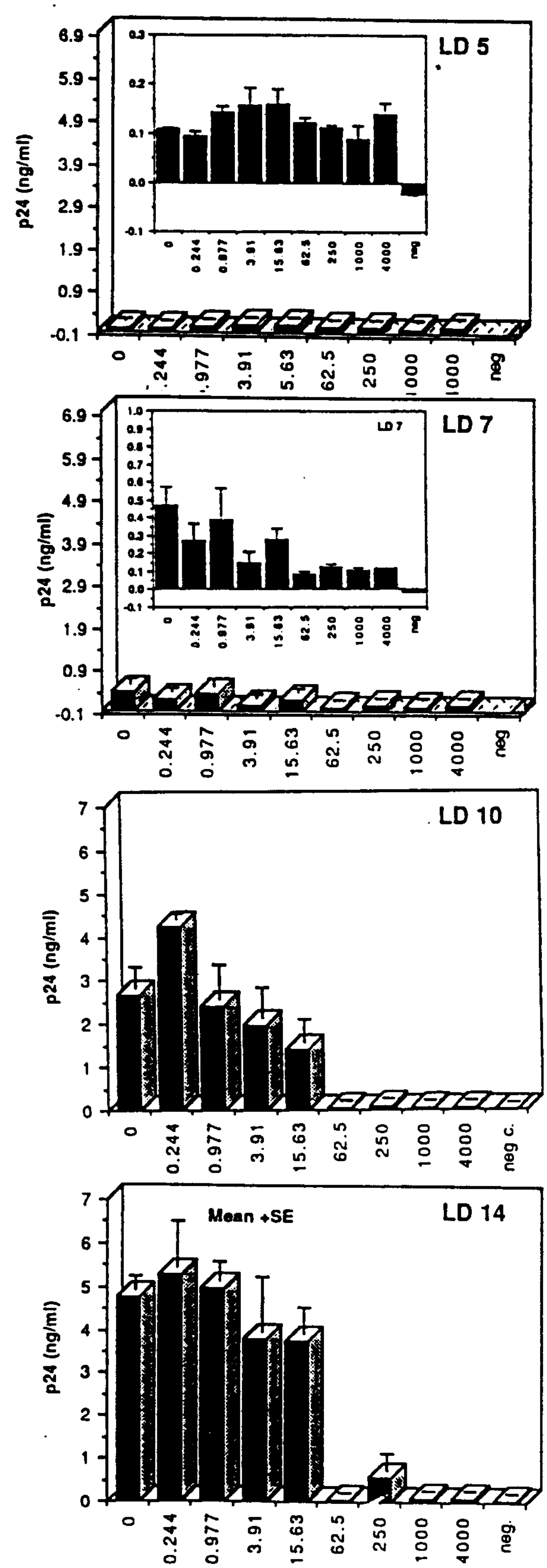


DCT (nM)

ddCTP

FIG. 4

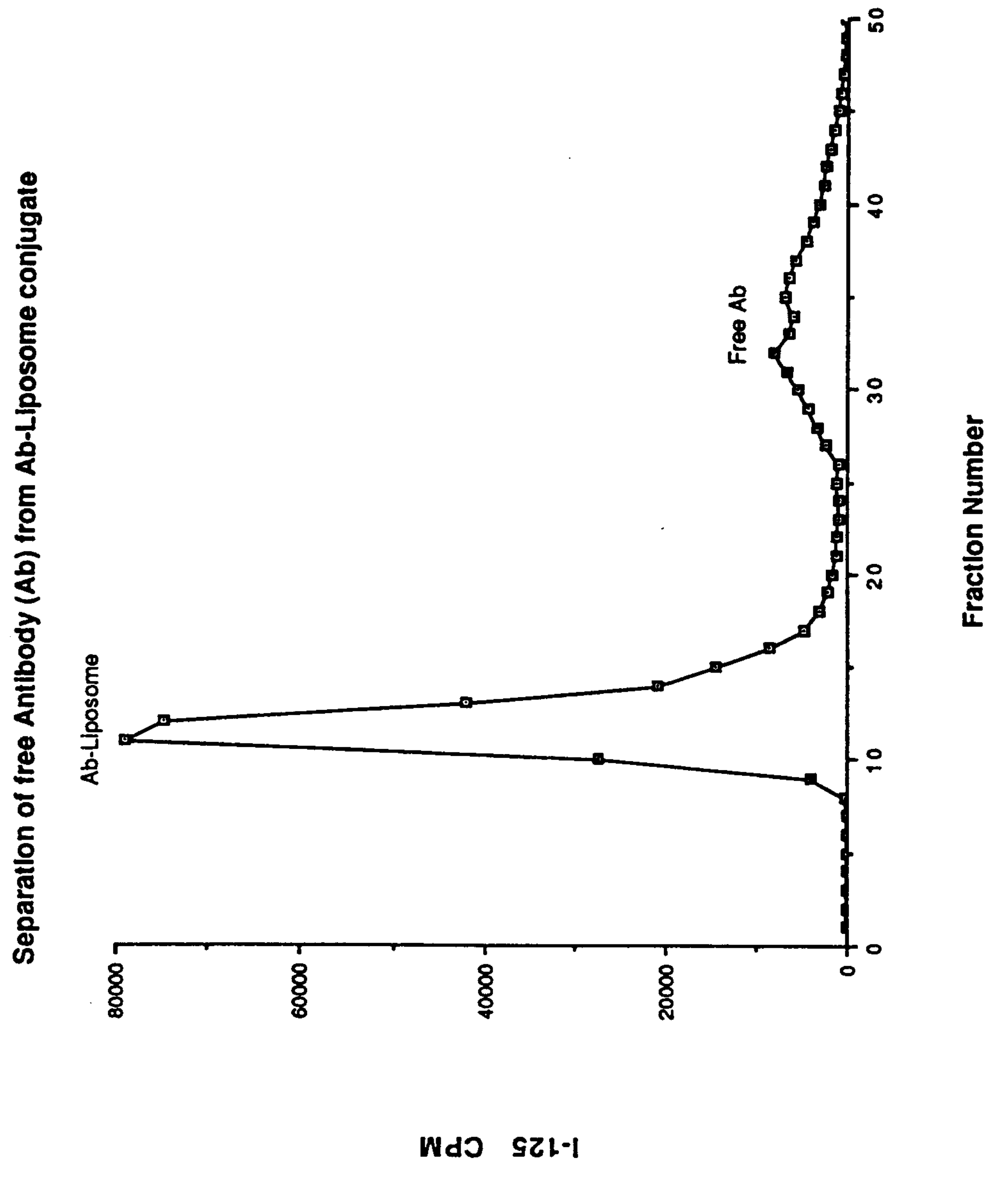
Separation of free Antibody (Ab) from Ab-Liposome conjugate
Ab-Liposome
Free Ab
I-125 CPM
80000
60000
40000
20000
0
0
10
20
30
40
50
Fraction Number

FIG.5